# EUROPEAN PATENT APPLICATION

(11) **EP 2 071 976 A2**
(43) Date of publication of application: **24.06.2009**
(21) Application number: 08171706.8
(22) Date of filing: 15.12.2008
(51) Int. Cl.: A45D 40/00, A61K 8/49, A61Q 15/00

(54) **Cosmetic sticks comprising labile active**

(30) Priority: 20.12.2007 EP 07150235
(71) Applicant: Unilever PLC, 100 Victoria Embankment London, Greater London EC4Y 0DY (GB); Unilever N.V., 3013 AL Rotterdam (NL)
(72) Inventor: BATCHELOR, Stephen, Norman, Bebington, Wirral, Merseyside CH63 3JW (GB); WILLIAMS, Jason, Richard, Bebington, Wirral, Merseyside CH63 3JW (GB)
(74) Representative: Pearce, Timothy

(57) **Abstract**

The photo-destruction of an ingredient that is susceptible to photo-destruction in a stick cosmetic product can be retarded by controlling the ingress of air through the base of the dispenser of that product. The invention is particularly suitable for antiperspirant compositions containing palmatine disposed within a bottom-fill dispenser.

## Description

The present invention relates to cosmetic products particularly to anhydrous cosmetic products and more particularly to products for contact application.

Cosmetic products for topical application to human skin comprise a cosmetic composition housed within a dispenser. For many products, the choice of dispenser is at the discretion of the producer of the product, and independent of the composition, provided that it satisfies generic considerations. Thus, for example, generally if the composition is in the form of firm stick, for dispensing from a stick dispenser, the composition needs to satisfy the generic criteria that it remains solid and integral at both use and storage temperatures. Choices as to shape of the dispenser and any mechanisms for dispensing the stick composition were made on the basis of the dispenser itself and did not take the composition into account. By the same token, when selecting ingredients for a stick composition, the selection has been made on the basis of the properties of the ingredients themselves and their interaction within the composition without taking into account the dispenser from which they are dispensed.

However, it has now been found that in some circumstances, it is advantageous to select the composition and the dispenser together, for example in order to overcome or at least ameliorate the instability of one or more ingredients in the composition. In particular, it has been found that the stability of certain ingredients in cosmetic formulations can be improved by control of air ingress.

The cosmetics industry has either proposed or adopted several different containers for cosmetic products ranging from a simple tub in which its contents is typically dispensed by extraction of the contents on a finger, through to more complicated dispensers in which the contents is dispensed by an advancing platform/piston/elevator pushing a stick through an opposed opening, coupled with a threaded spindle advancing mechanism. Herein, the term "stick" dispenser indicates that the dispenser comprises both a platform and a screw-threaded advance mechanism. The simple tub is simple to seal, whereas the stick dispenser is much more difficult, and especially when the platform itself comprises one or more channels through which the dispenser can be filled through its base.

WO 2007029187 discloses compositions, including solid compositions, containing a material that the instant inventors have recognised to be susceptible to UV/photo-destruction during storage, but the text neither recognises that the material suffers from such susceptibility nor identifies a way to improve the storage stability of the compound in compositions containing it when dispensed from a stick dispenser.

WO20040219122 discloses polyol in silicone emulsion compositions containing many different ingredients, but does not specify materials that are susceptible to UV/photo-destruction, nor does it contemplate incorporation of such materials in a stick dispenser, but merely an air tight container with no explanation.

WO2005055964 discloses on page 21 an antiperspirant composition containing an antiperspirant composition containing an encapsulated fragrance disposed within an unspecified dispenser. There is no disclosure of a material that is susceptible to UV/photo-destruction nor any indication of the nature of the dispenser and accordingly provides no teaching as to whether any dispenser would or would not be capable of improving the storage stability of such a susceptible material.

WO20020699924 discloses anhydrous antiperspirant compositions, including stick compositions on page 15, but does not contemplate expressly the presence of materials that are susceptible to UV/photo-destruction. The stick composition is contained in a conventional antiperspirant stick package. Accordingly, the disclosure does not provide any teaching to the reader as to how to select a stick dispenser that would ameliorate or overcome the problem of impaired storage stability of materials susceptible to UV/photo-destruction.

US4232977 discloses a dispenser for a stick composition that it alleges comprises a vapour tight seal (column 6, 3^{rd} paragraph). Unfortunately, it is the experience of Applicants that, notwithstanding the desirability of providing a seal that is vapour-tight, it is not possible in practice to do so when employing dispensers moulded from thermoplastics, and especially if the sealing system comprises but a single seal. The seal has to be loose enough to permit rotation of the shaft relative to the dispenser barrel, so that the sealing pressure of the seal is quite low so that several factors contribute to impairing the seal, in practice rather than in theory. First, there is commonly some variation in the size of individual components during the moulding operation and secondly, there can be imperfections in any seal at its periphery arising during the moulding or possibly damage occurring after moulding during transportation before assembly. Thirdly, during assembly, there can be minor, localised damage to the seal as the shaft bearing the seal is inserted through an aperture in the base of the dispenser barrel. All of these factors contribute to the concept of a vapour tight being theoretical. Moreover the disclosure in this specification does not contemplate any relationship between the extent to which a dispenser can seal at its base and it's applicability to compositions containing a material that is susceptible to UV/photo-destruction.

The disclosure in US2840231 in regard to the seal is similar to that of US4232977, and is similarly deficient in relation to the concept of the instant invention, namely selecting a dispenser meeting a particular criterion in conjunction with the incorporation of an ingredient having a particular property.

The disclosure in EP0462925 relates to sealing a cap, rather than at the base of a stick dispenser and does not contemplate any relationship between the dispenser and UV/photo-sensitive ingredients.

EP0070257 discloses a sealing means comprising multiple flanges but there is no disclosure of any relationship with an ingredient that is susceptible to UV/photo destruction and in particular no indication that by appropriate selection of a stick dispenser, it is possible to affect the storage stability of such an ingredient.

EP0189521 contemplates a dispenser lacking a screw-threaded advance mechanism and is silent concerning any relationship between the dispenser selection and an ingredient that is susceptible to UV/photo destruction.

The abstract of JP2005-281284 discloses compositions containing palmatine, but provides no direction to the reader as to how to improve its stability during storage.

### Brief summary of the present invention

According to one aspect of the present invention, there is provided a cosmetic product which is a composition disposed within a dispenser, in which the composition is in the form of a solid anhydrous stick composition containing one or more ingredients that are susceptible to photo-destruction by visible/UV light and/or UV light and the dispenser comprises a barrel having opposed first and second ends, the first end being closed by a cap and the second end comprising a base, a platform intermediate between the first and second ends and screw-threaded means for advancing the platform towards the first end mounted on or adjacent to the second end, the composition being disposed between the platform and the first end of the barrel, in which the dispenser permits a % weight gain after 21 days in a test procedure as defined herein of not greater than 150% of the % weight gain obtained in a reference dispenser.

By employing in conjunction such a composition and dispenser that satisfies the test, it is possible to at least ameliorate the instability of one of more of such ingredients in such a stick composition. The prior art as summarised hereinbefore shows that separately one or more compositions containing such an ingredient may have been disclosed hitherto, and that one or more dispensers that allegedly, but disputably, have been described that, if selected, might be suitable for the invention to be performed. However, separate disclosure of elements of an invention does not direct the reader or user to simultaneously make the two selections simultaneously, and particularly does not direct the reader or user to make such selections simultaneously in order to improve the stability of that ingredient.

The invention is particularly relevant for the selection of combinations in which the dispenser is bottom filled.

The relative weight gain is measured by the test procedure described herein.

According to a second aspect of the present invention, there is provided a method for lengthening the period during which a compound that is susceptible to photo-destruction remains effective in a solid composition which comprises in a first stage identifying its susceptibility to UV or photo-destruction, in a second stage incorporating it into a cosmetic composition, and thereafter introducing the composition into a dispenser comprising a barrel having opposed first and second ends, the first end being closed by a cap and the second end comprising a base, a platform intermediate between the first and second ends and screw-threaded means for advancing the platform towards the first end mounted on or adjacent to the second end, in between the platform and the first end of the barrel, in which the dispenser permits a % weight gain after 21 days in the test procedure as defined herein of not greater than 150% of the % weight gain obtained in the reference dispenser.

In a third aspect of the present invention there is provided a cosmetic product according to the first aspect that is an antiperspirant or deodorant product in which the cosmetic active comprises an antiperspirant or deodorant active.

### A more detailed description of the invention, including preferred embodiments.

The present invention is concerned with stick products displaying improved stability during exposure to ambient air, in visible and UV light, and is especially applicable where the air is humid. Such conditions are prevalent in various rooms of most houses in which cosmetic products are commonly stored, such as in or adjacent to a bathroom or shower-room or in the vicinity of wash hand basins, for example in order to facilitate bodily application of such products shortly after cleansing. Where the cosmetic composition contains an active ingredient that suffers from photo-destruction, the effectiveness of that ingredient is progressively impaired with the passage of time unless measures are taken to retard, ideally to zero, the rate at which the ingredient is destroyed.

The instant invention is of particular applicability to counter degradation of ingredients which is caused or accelerated by water, such as is present in humid air.

The present invention is of relevance to a cosmetic composition which contains an ingredient that is affected adversely by storage of such a product until it is consumed, which can commonly last for up to several months. Visible and UV light herein is considered to comprise light having a wavelength of from 290 to 740 nm and particularly up to 700 nm. Accordingly, the invention is especially applicable in regard to products that may be exposed to sunlight, since it comprises light across the full spectrum from 290 to 740 nm and particularly up to 700 nm, albeit possibly of reduced intensity across a fraction of the spectrum where that sunlight has passed through glass, such as through a glass window.

The invention broadly contemplates two mechanisms in which visible/UV light such as particularly sunlight can cause destruction of compounds. One mechanism comprises direct absorption of light by the compound in question, leading to its excitation and reaction in that excited state. Without being restrictive, many destructive reactions can be classified as one or other of hydrogen abstraction, internal rearrangement or electron transfer reactions. Of these reactions, the most common is the classification of electron transfer. A second mechanism can be classified as sensitized destruction which proceeds as in the first mechanism by a compound being promoted to an excited state by absorption of light, but thereafter, the excited state is transformed into a reactive intermediate which reacts with a complementary compound. Although not restricted to the creation of such reactive intermediates, the two most commonly formed intermediates are free radicals and singlet oxygen.

Many substances considered susceptible to photo-destruction can alternatively be classified as Photolabile, by which is meant a substance which when dissolved in a solvent and exposed to simulated Florida sunlight degrades by more than 50 mol%.

The solvent may be selected from: water at pH 4-8, ethanol, methanol, acetonitrile, nonane, toluene, glycerol, or combinations thereof. The substance at the start of the test should have a maximum optical density between 290 and 700 or 740 nm, at a path length of 1cm, of 0.5-1. The test should be carried out with a 1 cm light path length.

The dissolved substance should be exposed to 13860 kJ/m² in the range 300-800nm of simulated Florida sunlight produced by a Xenon arc lamp in a "weatherometer". A suitable weatherometer for such tests is an Atlas Ci3000+ Weatherometer.

Degradation of the substance may be monitored by UV-VIS spectroscopy or by chromatographic extraction and quantification of the substance.
(1) Molecules which produce singlet oxygen under exposure to light in the range 290-700 or 740nm and oxygen with a quantum yield greater than 0.05 in an organic solvent (methanol, acetonitrile, benzene, toluene, dimethylformide, CCl₄) or D₂O.
   Quantum yields for photosensitized formation of singlet oxygen may be found in J.Phys.Chem.Ref. Data 1993, vol 22, nol pp113-262.
   Examples of such substances include chlorophyll, coumarin, porphyrins, porphins, myoglobin, riboflavin, bilirubin, and methylene blue, xanthene based dyes.
(2) Molecules comprising aromatic heterocycles, and particularly when the heteroatom is charged. Desirable examples include palmatine or malvidin chloride. Other counter-ions can be employed if desired. 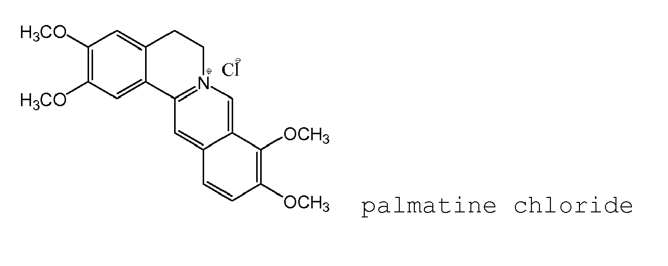 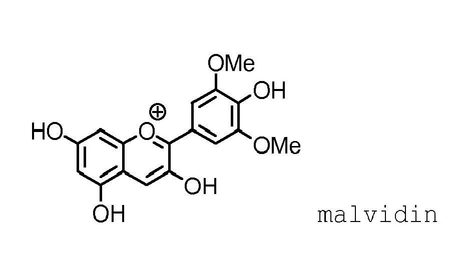
(3) Phenols which lack a tertiary butyl group that would serve to stabilise the phenol, adjacent to the OH group. The phenols are preferably polyphenols, such as catechin epicatechin, epicatechin gallate, epigallocatechin gallate resorcinol, gallic acid, isolquiritin 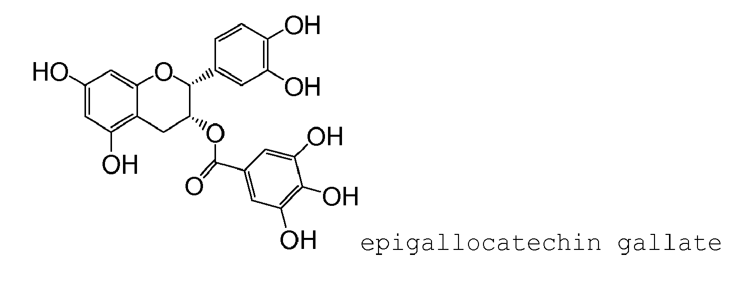
(4) biological molecules: Vitamins, such as vitamin C, vitamin B3 and/or derivatives thereof, Co-enzymes, Enzymes and proteins
(5) Unsaturated aliphatic olefinically unsaturated fatty carboxylic acids comprising a C-H bond alpha to the olefinic unsaturation;

Such compounds are contemplated for incorporation into cosmetic products on account of their beneficial properties. Thus, for example, various of them, such as dyes, can colour the composition rendering it attractive to a consumer.

Others such as C18 unsaturated carboxylic acids, can combat irritation on skin induced by co-ingredients. Yet others, such as vitamins, can act as antioxidants and thereby protect the skin from harmful external challenges. Still others can improve or disguise the appearance of hair on skin, such as by retarding its growth, either shorter and/or finer.

It is particularly desirable in some embodiments to employ a substance in accordance with class (1). In other embodiments, it is of benefit to employ a substance in accordance with class (2). In still other embodiments, it is desirable to employ a substance in accordance with class (3).

If desired, a mixture of two or more such substances from classes (1) to (5) can be employed together, selected from within a class or from different classes.

In relation to antiperspirant or deodorant products, it is particularly desirable to employ compounds, such as those in the classes mentioned above, that improve skin condition or counter skin aging or combat irritation, or act as antioxidants or retard hair growth.

An ingredient is considered to be adversely affected if the rate of impairment of a desired property is accelerated or the extent of impairment increased by storage in daylight in ambient air compared with storage in the dark in air of zero percent relative humidity. Many such ingredients are expressly recognised from their data sheets to be susceptible to photo-destruction, by reference to their extinction coefficient having a peak value within the visible/UV light spectrum of at least 100 mol⁻¹cm⁻¹, particularly at least 500 mol⁻¹cm⁻¹ and especially at least 1000 mol⁻¹cm⁻¹. For the others, their susceptibility can be assessed as indicated above, or indeed their extinction coefficient can be measured using a conventional test employing a standard cell such as of 1cm width and a solution of the compound in a compatible solvent at a known concentration through which light is shone, and the net extent of absorption measured across the spectrum (after allowance for the solvent alone).

It will be understood that, herein, the "peak value" is the highest value for the extinction coefficient within the spectrum of from 290 nm to 740nm. In the event that the value for the extinction coefficient continues to increase through the boundary of said spectrum, then the wavelength for the peak is taken to be at the spectrum boundary itself, that is to say 290 nm or 740 nm as the case may be.

It will further be recognised that certain, but not all, ingredients indicated above which are potentially susceptible to photo-destruction contain a chromophore.

The concentration of the ingredient susceptible to photo-destruction is normally at least 0.0001% w/w of the composition, in many embodiments at least 0.0005% w/w and in some preferred embodiments, at least 0.001%. Said ingredient is commonly present at a concentration not exceeding 10% w/w, and often not higher than 5% w/w of the composition. In a number of highly desirable compositions, for example those containing a susceptible ingredient affording skin care or hair minimisation benefit, the ingredient is present at a concentration of up to 1% w/w.

For the avoidance of doubt, an astringent antiperspirant active, such as salts described hereinafter, is considered not to be an ingredient affected adversely by such storage.

Products herein comprise an anhydrous composition, by which is meant that the composition is free from detectable free water. Free water excludes water of hydration.

The dispenser for the stick material preferably demonstrates a weight increase after 21 days in the test procedure of not greater than 125% and particularly not greater that 110% of the weight gain in the primary reference dispenser, and especially at or less than in the primary reference dispenser.

### Test Procedure for assessing suitability of a dispenser.

Herein, in the test procedure, the reference and the trial dispensers are each filled with approximately a 50g sample of the stick composition specified hereinbelow, with the stick "former" removed if the dispenser has been bottom filled, and a close-fitting cap is fitted over the open end. Bottom filling, as is conventional, means that the stick composition is filled into the dispenser through an aperture at the base of the dispenser and through one or more passages in the platform, the composition being retained in the dispenser barrel between the platform and the first end of the barrel. In respect of dispensers employing a screw-threaded mechanism for advancing the platform, dispensers which include the reference dispenser, the mechanism for platform advancement is mounted on the barrel, and any seal fitting in or covering a filling aperture. The sample and reference dispenser are filled such that the stick material occupies the entire volume between the platform and the open end of the barrel.

The filled sample and reference dispensers are weighed and then stood upright side by side in a humid domed chamber having a diameter of 30 cm and a height of 25cm which contains a bowl of deionised water which humidifies the air within the chamber to greater than 90% RH at 23°C, and stored in a room at laboratory ambient temperature, the room typically reaching about 22-23°C during the day, but often falling to below 20°C overnight. The test procedure measures the relative weight gain of the products containing the same reference composition identified below to identify the relative effectiveness of the dispenser at controlling air ingress. Although variation in the absolute rate of weight gain may occur as a result of for example fluctuation in the temperature, any variation in the relative humidity within the chamber remains within the range of at least 90% RH, and any such absolute variations apply to both sample and reference products simultaneously, the comparison using the same defined composition, so that the relative weight gain is not affected.

At intervals, preferably weekly, the dispensers are weighed and replaced in the chamber, the difference in weight being measured. For the purpose of determining whether or not the sample dispenser is in accordance with the present invention, the weight increase comparison is made after 21 days, namely on the 22^{nd} day of the test. The weighing on the 22^{nd} day should take place at substantially the same time of day as the initial weighing. Preferably, the data is collected on at least 5 dispensers for each of the reference and sample dispensers, and averaged. Preferably, the trial is conducted using dispensers that have not been filled previously. Primary reference dispensers expressly and solely for the purpose of conducting the test procedure, and in numbers sufficient for that purpose, are held by and available from Unilever Research & Development, Quarry Road East, Bebington, CH63 3JW, England, at the cost of the requester. Any such request for reference dispensers shall be marked "Kenzo Reference Dispenser, 1.6oz Dove White Meteor".

The test preferably employs the same weight, about 45g of the reference stick material in each dispenser. The reference dispenser is a nominal 1.6 oz dispenser that accommodates about 45g material. As is described in more detail hereinafter, the reference dispenser is bottom filled through a central opening within the rotor wheel mounted through a central aperture in the base of the dispenser barrel and thence though passageways in the platform. The platform is skeletal. The dispenser comprises essentially two orthogonal seals sealing the rotor wheel into the aperture at the base of the barrel.

For comparison with markedly different weights of stick material in the reference and test dispensers, the average weight gain should be converted to an average gain per 100g of stick material.

Such reference dispensers constitute the primary reference dispenser. If, as may happen eventually, a supply of the primary reference dispenser is exhausted, then a secondary reference dispenser can be supplied instead, namely one that has been referenced to the primary reference dispenser, together with the conversion factor to determine whether or not the invention test is met. Thus, if the secondary reference dispenser permits a weight increase that is "x" times the weight increase of the primary reference dispenser, then the appropriate ceiling ratio to determine whether or not a sample dispenser satisfies the instant invention is 1.5/x. Such conversion factor shall be supplied together with the secondary reference dispenser.

The stick composition for conducting the above assessment of the suitability of a dispenser is as follows:-

| **Chemical Name** | **Trade name** | **Supplier** | **% w/w** |
|---|---|---|---|
| Cyclopentasiloxane | DC245 | Dow Corning | 27.00 |
| PPG-14 Butyl Ether | Fluid AP | Amerchol | 9.50 |
| Butylhydroxyltoluene | BHT, 100% Active | Eastman | 0.05 |
| Dimethicone | DC200/ 50cst | Dow Corning | 1.00 |
| C₁₂-₁₅ Alkyl Benzoate | Finsolv TN | Finetex | 15.00 |
| Steareth-100 | Brij 700 | Uniqema | 0.50 |
| Stearyl Alcohol | Lanette C18 Deo | Cognis | 18.00 |
| Hydrogenated Castor oil | Castor Wax MP-80 | Caschem | 3.50 |
| Polyethylene wax | Performalene 400 | | 1.00 |
| PEG-8 | Polyethylene glycol 400 | Fluka | 2.00 |
| Fumed silica | Aerosil 200 | Degussa | 0.75 |
| Aluminium Chlorohydrate | AACH- A418 | Summit | 20.00 |
| Sunflower Oil | Agripure 80 | Cargill BV | 0.50 |
| Fragrance | | | 1.20 |

The product according to the present invention comprises at least one cosmetic active ingredient, for example one or more that can impart colour, such as a pigment, inhibit body odour generation, inhibit perspiration, impart moisturisation, combat ageing of skin, condition skin or hair, protect skin against exposure to sunshine and/or ameliorate sunburn or repel insects.

The invention product especially convenient comprises an antiperspirant or deodorant active.

Antiperspirant actives for use herein are often selected from astringent active salts, including in particular astringent aluminium, zirconium and mixed aluminium/zirconium salts, including both inorganic salts, salts with organic anions and complexes. Preferred astringent salts include aluminium, zirconium and aluminium/zirconium halohydrate salts, and especially chlorohydrates. Aluminium/zirconium chlorohydrates complexed with glycine are particularly desirable antiperspirant actives in stick compositions herein.

Aluminium halohydrates are usually defined by the general formula Al₂(OH)ₓQ_{y}.wH₂0 in which Q represents chlorine, bromine or iodine, x is variable from 2 to 5 and x + y = 6 while wH₂O represents a variable amount of hydration. Aluminium chlorohydrates comprise a mixture of polymeric species, for example species containing respectively 12, 24 or 36 aluminium atoms and the relative ratio of the various species is controlled by the manufacture process. It is desirable to include a high proportion of A1 24 species, such as products obtained following the teaching in EP-A-6739, sometimes called activated aluminium chlorohydrates.

Zirconium actives can usually be represented by the empirical general formula: ZrO(OH)_{2n-nz}B_{z}.wH₂0 in which z is a variable in the range of from 0.9 to 2.0 so that the value 2n-nz is zero or positive, n is the valency of B, and B is selected from the group consisting of chloride, other halide, sulphamate, sulphate and mixtures thereof. Possible hydration to a variable extent is represented by wH₂0. Preferable is that B represents chloride and the variable z lies in the range from 1.5 to 1.87. In practice, such zirconium salts are usually not employed by themselves, but as a component of a combined aluminium and zirconium-based antiperspirant.

The above aluminium and zirconium salts may have coordinated and/or bound water in various quantities and/or may be present as polymeric species, mixtures or complexes. In particular, zirconium hydroxy salts often represent a range of salts having various amounts of the hydroxy group. Zirconium aluminium chlorohydrates may be particularly preferred.

Antiperspirant complexes based on the above-mentioned astringent aluminium and/or zirconium salts can be employed. The complex often employs a compound with a carboxylate group, and advantageously this is an amino acid. Examples of suitable amino acids include dl-tryptophan, dl-β-phenylalanine, dl-valine, dl-methionine and β-alanine, and preferably glycine which has the formula CH₂ (NH₂) COOH. It is highly desirable to employ glycine complexes of a combination of aluminium halohydrates and zirconium chlorohydrates together with amino acids such as glycine.

Many suitable antiperspirant salts have an Al/Zr ratio in a range from 2 to 10, especially 2 to 6, a metal to Cl ratio from 2.1 to 0.9:1 and a variable amount of glycine. In some highly desirable complexes, the metal:Cl ratio is from 0.9:1 to 1.25:1 and in others it is from 1.3:1 to 1.45:1.

Other actives which may be utilised include astringent titanium salts, for example those described in GB 2299506A.

The proportion of solid antiperspirant salt in a suspension composition normally includes the weight of any water of hydration and any complexing agent that may also be present in the solid active.

The antiperspirant salt in an anhydrous stick composition is particulate, commonly having particles mainly with a diameter within the range of 0.1 to 200 µm, such as providing a mean particle size in the range of from 3 to 20µm.

The particulate antiperspirant active may be present in the form of hollow spheres or/and non-hollow particles at the discretion of the manufacturer of the invention antiperspirant product. Non-hollow particles can be made, if desired, by crushing hollow particles. Where it is desired that the composition is translucent in bulk or to reduce the appearance of visible deposits on the skin to which the composition is applied or on clothing which comes into contact with the composition, it is preferable for the antiperspirant particles to be substantially free from hollows, such as greater than 90% by weight of the particles and especially greater than 95% by weight.

The antiperspirant active is often present at a concentration of from 0.1 to 35% by weight of a stick composition, particularly at least 5% by weight and in many very desirable compositions at least 15% by weight. Often, its concentration is not greater than 30% by weight, and in many effective compositions is up to 26% by weight. At low concentrations such as up to 5% by weight, the active is more noticeable as a deodorant, whereas at the higher concentrations and especially at above 10% concentration, it increasingly demonstrates effectiveness to reduce perspiration whilst retaining its ability to inhibit malodour formation.

Suitable deodorant actives can comprise deodorant effective concentrations of antiperspirant metal salts, deoperfumes, and/or microbicides, including particularly bactericides, such as chlorinated aromatics, including biguanide derivatives, of which triclosan (eg Irgasan DP300 or Triclorban), and chlorhexidine warrant specific mention. Another class of effective deodorants comprises polyaminopropyl biguanide salts such as are available under the trade mark Cosmosil. Such materials commonly act as bactericides. A still further class of materials that can inhibit malodour formation comprise chelators that can sequester iron, and thereby retard bacterial growth, including aminopolycarboxylates such as EDTA or higher homologues such as DTPA. Deodorant actives other than astringent metal antiperspirant salts are commonly employed at a concentration of from 0.1 to 5% by weight, and particularly 0.1 to 2% by weight.

Compositions herein commonly comprise one or more carrier liquids, which are water-immiscible and thus sometimes are referred to as oils. The liquids often constitute from 30 to 90% by weight of the composition and in many desirable compositions at least 40 % and particularly at least 50% by weight. Their total weight proportion is often not greater than 80% and in many practical embodiments of the invention is up to 70%.

The carrier liquids often include silicone oils, which may be volatile or non volatile or a mixture of both and commonly also or alternatively comprise non-silicone oils.

It is preferred that the hydrophobic carrier liquids include a volatile liquid silicone, i.e. liquid polyorganosiloxane. To class as "volatile" such material should have a measurable vapour pressure at 20 or 25°C. Typically the vapour pressure of a volatile silicone lies in a range from 1 or 10 Pa to 2 kPa at 25°C.

It is desirable to include a volatile silicone because it can give a "drier" feel to the applied film after the composition is applied to skin, for example constituting at least 25% and particularly at least 40% by weight of the carrier liquids.

Volatile polyorganosiloxanes can be linear or cyclic or mixtures thereof. Preferred cyclic siloxanes include polydimethylsiloxanes and particularly those containing from 3 to 9 silicon atoms and preferably not more than 7 silicon atoms and most preferably from 4 to 6 silicon atoms, otherwise often referred to as cyclomethicones. Preferred linear siloxanes include polydimethylsiloxanes containing from 3 to 9 silicon atoms. The volatile siloxanes normally by themselves exhibit viscosities of below 10⁻⁵ m²/sec (10 centistokes), and particularly above 10⁻⁷M²/sec (0.1 centistokes), the linear siloxanes normally exhibiting a viscosity of below 5 x 10⁻⁶m²/sec (5 centistokes). The volatile silicones can also comprise branched linear or cyclic siloxanes such as the aforementioned linear or cyclic siloxanes substituted by one or more pendant -0-Si(CH₃)₃ groups. Examples of commercially available silicone oils include oils having grade designations 344, 345, 244, 245 and 246 from Dow Corning Corporation; Silicone 7207™ and Silicone 7158™ from Union Carbide Corporation; and SF1202™ from General Electric.

The hydrophobic carrier employed in compositions herein can alternatively or additionally comprise non-volatile silicone oils, which include polyalkyl siloxanes, polyalkylaryl siloxanes and polyethersiloxane copolymers. These can suitably be selected from dimethicone and dimethicone copolyols. Commercially available non-volatile silicone oils include products available under the trademarks Dow Corning 556 and Dow Corning 200 series. Other non volatile silicone oils include that bearing the trademark DC704. Incorporation of at least some non-volatile silicone oil having a high refractive index such as of above 1.5, for example in a proportion of at least 10% by weight of the silicone oils can be beneficial in some compositions.

Silicone oils may be supplemented, if desired, by other oils, and in such instances, there is preferably, sufficient liquid silicone to provide at least 10%, better at least 15%, by weight of the whole composition.

Silicon-free hydrophobic liquids can be used, preferably in addition to liquid silicones. Silicon-free hydrophobic organic liquids which can be incorporated include liquid aliphatic hydrocarbons such as mineral oils or hydrogenated polyisobutene, often selected to exhibit a low viscosity. Further examples of liquid hydrocarbons are polydecene and paraffins and isoparaffins of at least 10 carbon atoms. Hydrocarbon liquids preferably are present in a range of from 0 to 20% w/w and especially from 0 to 5% of the oils.

Other suitable hydrophobic carriers comprise liquid aliphatic or aromatic esters. Suitable aliphatic esters contain at least one long chain alkyl group, such as esters derived from C₁ to C₂₀ alkanols esterified with a C₈ to C₂₂ alkanoic acid or C₆ to C₁₀ alkanedioic acid. The alkanol and acid moieties or mixtures thereof are preferably selected such that they each have a melting point of below 20°C.
These esters include isopropyl myristate, lauryl myristate, isopropyl palmitate, diisopropyl sebacate and diisopropyl adipate.

Suitable liquid aromatic esters, preferably having a melting point of below 20°C, include fatty alkyl benzoates. Examples of such esters include suitable C₈ to C₁₈ alkyl benzoates or mixtures thereof, including in particular C₁₂ to C₁₅ alkyl benzoates eg those available under the trademark Finsolv.

Ester oils, be they aliphatic or aromatic desirably comprise from 0 to 50% w/w, eg 5 to 40% w/w of the oils.

Yet other suitable ester oils, which desirably provide up to 10% w/w of the carrier liquids or alternatively from 1 to 6% w/w of the composition comprise triglyceride oils, such as those extractable from plants derivable from unsaturated carboxylic acids, such as C16, C18 and/or C20. Suitable examples of such triglyceride oils include caster oil, borage oil, coriander seed oil, safflower oil and sunflower seed oil.

Further instances of suitable hydrophobic carriers comprise liquid aliphatic ethers derived from at least one fatty alcohol, such as myristyl ether derivatives e.g. PPG-3 myristyl ether or lower alkyl ethers of polygylcols such as an ether having named as PPG-14 butyl ether by the CTFA. Such ethers desirably constitute from 0 to 20, and preferably from 0 to 10% w/w of the oils.

Yet other oils which can beneficially be included comprise hydrophobic aliphatic alcohols which are liquid at 20°C and have a boiling point of above 100°. Such oils are preferably employed in a proportion of from 0 to 50% w/w of the carrier liquids, and are of especial benefit for use in conjunction with amido gellants, when they are preferably employed within the proportion of from 25 to 50% w/w of the carrier oils. Especially desirable hydrophobic aliphatic alcohols are branched chain alcohols of at least 15 carbon atoms up to 30 and especially up to 25, including isostearyl alcohol, hexyl-decanol octyl-dodecanol and decyl-tetradecanol. Other suitable water-immiscible alcohols include intermediate chain length linear alcohols, commonly containing from 9 to 13 carbon atoms, such as decanol or dodecanol. A further suitable alcohol is benzyl alcohol.

Stick compositions herein normally comprise a structurant or gellant for the carrier liquid.

One class of structurant which is desirable by virtue of its long standing proven capability to produce firm solids (sticks) comprises waxes. Herein, the term wax is employed to encompass not only materials of natural origin that are solid with a waxy feel and water-insoluble at 30-40°C, but melt at a somewhat higher temperature, typically between 60 and 95°C, such as beeswax, candelilla or carnauba wax and their synthetic derivatives or analogues for example those available from Koster Keunen under the marks K62, K67, or K82, but also materials having similar waxy properties.
Such other waxes include hydrocarbon waxes, eg paraffin wax, mineral wax and microcrystalline wax; synthetic waxes, such as polyethylene waxes of 300 to 600 daltons; waxy derivatives or waxy components of natural waxes, such as ester components, either extracted or synthesised, solid ester derivatives of glycerol or glycol, typically with linear saturated fatty acids, usually containing a significant fraction of C₁₆₋₂₂ acid residues, which may be synthesised or obtained by hydrogenating the corresponding natural oil, such as caster wax; petroleum waxes, waxy silicone polymers containing alkyl substituents of at least C₁₀ chain length; and, importantly, waxy fatty alcohols, that normally are linear and often contain from 14 to 24 carbons, such as stearyl alcohol, cetyl alcohol and/or behenyl alcohol.

Waxes are commonly employed at an amount of at least 12% by weight of the carrier oils, such as in the range of from 15 to 40% w/w and in many instances from 20 to 35% w/w. When considering the entire composition, the wax or mixture of waxes often constitute from 12 to 24% w/w of the composition, and in many compositions from 14 to 20% w/w.

A second class of structurants comprises oil-soluble copolymers of styrene and a short chain alkylene diene like butadiene, sometimes referred to as SEBS copolymers, obtainable under the trade mark Kraton. Other suitable polymeric structurants include copolymers of silicone and polyamides. Polymeric structurants in this second class can typically be employed at a concentration of from 3 to 12% w/w of the carrier oil blend.

A third class of structurant comprises non-polymeric materials that form a network of fibres within an oil phase, it is surmised by individual molecules or pairs of molecules stacking one above the other in an ordered fashion. One example is hydroxystearic acid. Many of such structurants comprise amide or ester links within the molecule. Examples of such structurants containing ester groups include α cellobiose octanonanoate and related compounds, and β maltose octatetradecanoate. Other materials include, in combination β sitosterol and γ oryzinol. Other examples include (S,S)-1,4-Di-O-Benzyl-D-Threitol and related compounds, such as disclosed in USP 6410001. Examples of desirable amide-containing fibre-forming structurants include 1,2- or 1,3- bisalkylamido cyclohexanes as described in USP 6410003, hydroxystearic acid amides described in USP 5840286, alkylamide derivatives of succinic or citric acid described in USP 6190673, aspartame-based cyclodipeptides having aromatic or cycloaliphatic substitution, eg the thymol derivative, as described in EP 1465586, and N-acyl aminoacid dialkylamides such as N-Lauroylglutamic Acid Di-N-Butylamide and N-(2-ethyl-hexyloyl)glutamic Acid Di-N-Butylamide and related compounds described in respectively USP 36969087 and US2002/0159961. Structurants in this third class tend to be able to form gels at comparatively low concentrations, and particularly those comprising amide links. They are commonly contemplated in a w/w amount of from 1 to 20% of the oil blend, and in many favourable compositions from 3 to 10% of the oil blend. Expressed in terms of the entire composition, the amount of such structurants is often from 3 to 7% w/w of the composition.

Solid compositions herein can comprise, if desired, one or more humectants, preferably comprising at least 2 hydroxyl substituents. Preferred humectants comprise glycerol and PEG (polyethylene glycol) having an average molecular weight of from 200 to 620. Such a humectant can desirably be employed at a concentration of at least 0.25% w/w and particularly at least 0.5% w/w of the composition. The humectant is preferably present at a concentration of up to 10% w/w, in many instances up to 8% w/w, and often advantageously from 1 to 4% w/w of the composition.

The humectant can advantageously be incorporated in the stick compositions herein together with a triglyceride oil, particularly derived from unsaturated C₁₈ aliphatic acids. The weight ratio of these two constituents is desirably in the range of from 4:1 to 1:4, for example providing a combined weight of from 1.5 to 6% w/w of the composition.

The solid compositions herein can if desired comprise one or more minor ingredients that can be contemplated in cosmetic compositions. Such ingredients normally comprise in total not more than 10% by weight of the composition. Such optional constituents can comprise sensory modifiers, such as talc or finely divided polyethylene, such as in an amount of up to 5% by weight; fragrance, including, if desired deoperfumes, often in an amount of up to 4%, eg 0.3 to 2% by weight, colorants; skin cooling agents such as menthol; wash-off agents such as non-ionic surfactants.

The solid cosmetic compositions contemplated herein and containing an ingredient that is susceptible to photo-degradation can be made by methods hitherto contemplated for similar compositions from which the photodegradable ingredient is absent.

By way of example, in the context of making an antiperspirant composition, hitherto described methods can be followed. In one such method, a blend of oils is formed and heated to a temperature which is high enough to dissolve the structurant therein. This temperature is commonly in the range of from 70 to 95°C when employing a wax, and commonly in the range of 70 to 140°C when employing a polymer or fibre-forming structurant (classes 2 and 3, supra). The precise temperature to be attained depends on the structurant itself. The composition is often allowed to cool to a temperature below the structurant dissolution temperature, but above the composition solidification temperature and the antiperspirant and the photo-degradable ingredient and other and optional ingredients introduced with agitation to prevent settling. The fragrance is often the last material to be introduced. The resultant mixture is introduced into the dispenser whilst it is still mobile, for example at a temperature of at least 3°C above its normal solidification temperature if filling takes place under gravity, or within +/- 2°C of that temperature if it is injected under external pressure.

It is however at the discretion of the producer to introduce some or all of the ingredients into the oil blend before it is heated to structurant dissolution temperature. Likewise, it is an alternative to introduce some or all of the structurant in a proportion of the oil blend that is heated to dissolve the structurant and other ingredients together with the remainder of the oil blend having a lower temperature, and the two fractions brought together to form a mixture.

The dispenser for the invention product can be selected in accordance with the test procedure identified above. The primary reference dispenser itself is a particularly suitable dispenser for the present invention, because it controls extremely well the inflow of ambient air into its interior through its base. Ambient air, as mentioned hereinbefore, will be particularly humid in locations such as bath or shower rooms or adjacent to washing facilities. However, in tropical or sub-tropical climes, the air is naturally humid, often exceeding relative humidity of 70%. Even humidities prevalent in temperate climates such as 40 to 50% can be considered moist. The top of solid cosmetic dispensers are commonly equipped with their tightly fitting cap, often a friction fit, but the base of dispensers can offer a passage for air ingress, for example between the base itself and the mounting for the rotor. In the primary reference dispenser sealing is especially effective, by virtue of employing a combination of sealing surfaces and seals, orthogonal to each other. The use of twin seals offers a recognisable gain over use of a single seal, providing two locations where inflow is obstructed, but the benefit is compounded by employing them orthogonally. Dispenser bodies and rotors are commonly produced by mass production methods, moulding them from thermoplastic. Some minor variations between individual bodies and rotors is accordingly inevitable, so that the sealing system has to encounter slightly different spacings between the opposed surfaces of dispenser base and the rotor mounting that are bridged by the seals. Moreover, in use, rotation of the rotor will flex the various elements of the dispenser, exacerbating any gaps. Orthogonality of the seals inevitably requires the seals to be bridging two sets of opposed surfaces, the one set being orthogonal to the other. Thus, the risk is reduced of minor variations in manufacture and flexing in use resulting in greater air ingress.

Advantageously, the reference dispenser, and particularly if it is a bottom-filled dispenser, employs for at least one of its seals, a claw seal. This is an especially effective seal for preventing air ingress, by virtue of its tip flexibility.

The test procedure identified herein is particularly suitable for identifying and selecting those bottom-fill stick dispensers which are preferable for incorporating compositions containing an ingredient that is sensitive to photo-degradation. In such dispensers, there is a significant surface area of stick material that is exposed at the base of the platform and thus in contact with air that can ingress through the base of the dispenser. In particular, the test procedure can identify suitable dispensers in which the area of exposed passage at the lower side of the platform constitutes at least 30%, often at least 50% and particularly at least 60% of the transverse surface area of the dispenser barrel within which the platform fits, such as up to 90% of the transverse surface area, and in many practical embodiments up to 85% of the transverse surface area. In calculation the %, the central threaded interior aperture of the hub of the platform is excluded.

Having summarised the present invention and provided general or preferred embodiments, specific embodiments will now be described more fully by way of example only.

In these Examples, the composition summarised below was made by the following general method:-A blend of the oils formed with stirring in a pot was slowly heated to a temperature in the region of 80 to 85°C. The solid structurants were introduced slowly with stirring until by eye the structurants were not visible as a separate dispersed phase. The mixture was allowed to cool to between 70 and 75°C and the particulate antiperspirant and other materials were introduced, including an ingredient containing palmatine which is susceptible to photo-degradation, the mixture being stirred to prevent sedimentation. The final ingredient added was the fragrance. The suspension of particulates in an oil phase was allowed to cool further until it was between 5 and 10°C above its normal solidification temperature and thereafter poured into the chosen dispenser through a central aperture in its base, be it a dispenser satisfying the criterion identified herein or a comparison dispenser that does not satisfy the criterion. The resultant products were inverted after the composition had solidified and allowed to cool to laboratory ambient. The central aperture was plugged and the dispenser subsequently subjected to testing to determine the stability of the photo-sensitive ingredient.

| **Chemical Name** | **Trade name** | **Supplier** | **% w/w** |
|---|---|---|---|
| Cyclopentasiloxane | DC245 | Dow Corning | 26.0975 |
| PPG-14 Butyl Ether | Fluid AP | Amerchol | 9.50 |
| Butylhydroxyltoluene | BHT, 100% Active | Eastman | 0.05 |
| Dimethicone | DC200/ 50cst | Dow Corning | 1.00 |
| C₁₂₋₁₅ Alkyl Benzoate | Finsolv TN | Finetex | 15.00 |
| Steareth-100 | Brij 700 | Uniqema | 0.50 |
| Stearyl Alcohol | Lanette C18 Deo | Cognis | 17.40 |
| Hydrogenated Castor oil | Castor Wax MP-80 | Caschem | 3.50 |
| Polyethylene wax | Performalene 400 | | 1.00 |
| PEG-8 | Polyethylene glycol 400 | Fluka | 2.00 |
| Fumed silica | Aerosil 200 | Degussa | 0.75 |
| Aluminium Chlorohydrate | AACH- A418 | Summit | 20.00 |
| Sunflower Oil | Agripure 80 | Cargill BV | 0.50 |
| Palmatine | Depiline 2XC (0.16% active) | Sederma | 1.50 |
| Aluminium salt of monoazoic dye | Unipure LC324 | Sensient | 0.0025 |
| Fragrance | | | 1.20 |

Palmatine has an extinction coefficient that is highest of about 2700/Mcm at 280nm, so that for the purpose of this invention, the "peak" extinction coefficient is taken to be at 290nm.

The primary reference dispenser is described herein with reference to the accompanying figures in which

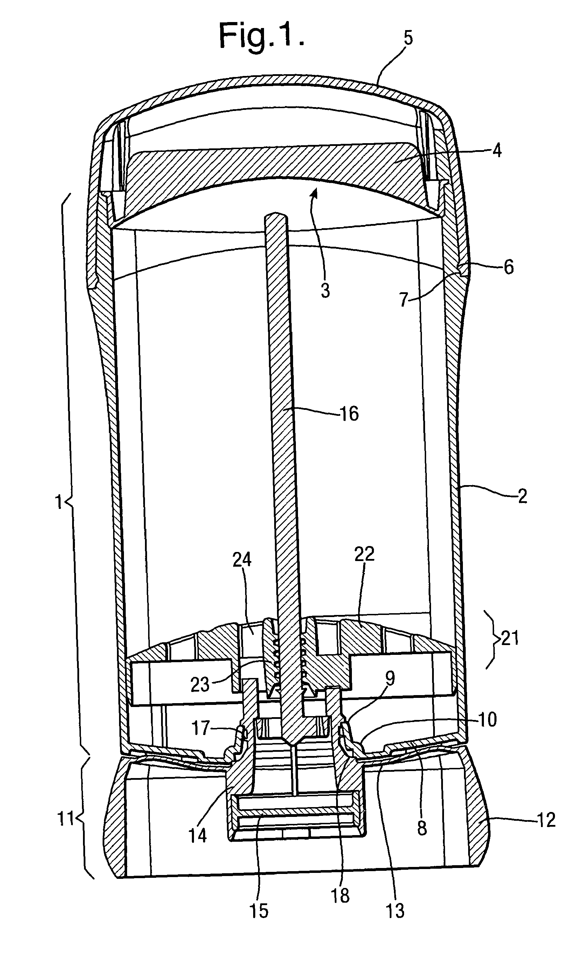

Fig 1 represents a cross section through the dispenser, viewed along its minor transverse axis;

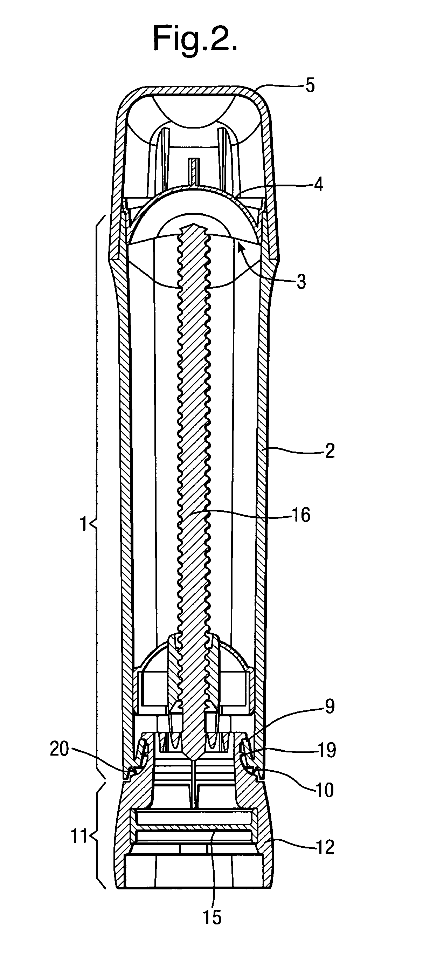

Fig 2 represents a cross section through the dispenser, viewed along its major transverse axis.

The dispenser comprised barrel (1) of oval cross section having a tubular axially extending sidewall (2) with an open upper end (3) in which is fitted a stick former (4) and which is covered by a cap (5) that friction fits onto the sidewall (2) and is held by a pair of short ribs and grooves (6), (7). Sidewall (2) has a base wall (8) having a central circular axial wall (9) joined to an annular lateral wall section (10). A full base rotor (11) sits beneath the barrel (1) and comprises a peripheral sidewall (12) and a lateral top wall (13) and a central tubular axial wall (14) dimensioned to be pushed partly through the axial circular wall (9) of the barrel base. Tubular wall (14) has a lower section forming an aperture plugged by plug (15) and from its upper section an axial threaded spindle (16) extends into the body of the barrel (1). Apertures in the mounting of the spindle (16) in the tubular wall (14) permit flow therethrough of stick material when it is in fluid form.
The tubular wall (14) comprises two external surfaces substantially orthogonal to each other (17, 18) that are opposed to the inward face of walls (9) and (10) respectively. Between the opposed faces of wall (9) and surface (17) extends a claw seal (19) and between wall (10) and surface (18) extends a wiper seal (20). A platform (21) is located within barrel (1) between its base wall (8) and its open end (3) having a domed top (22), a plurality of apertures (24) and a central threaded bore (23) dimensioned to engage with the threaded spindle (16).

The dispenser is made by push fitting the central wall (14) of the rotor (11) through the circular section (9) of the base and the seals (19) and (20) span respectively the spaces between surfaces (9, 17) and (10, 18). The platform (21) is fitted through open end (3) and the rotor (11) rotated to wind the platform down until it encounters the barrel base. The former (3) is push fitted and the dispenser is inverted. The barrel (1) is filled with stick material (not illustrated) through the apertures in the circular wall (14) and apertures (24) in the platform (21). When the stick material has solidified, the dispenser is turned upright and the cap fitted.

Said primary reference dispenser after 21 days had gained under the high humid test conditions specified hereinabove on average 0.0707g, the starting weight of the dispenser and stick material being 85.633g on average. The average weight gain per day was thus 0.003367g.

The above-summarised formulation containing the ingredient susceptible to photo-destruction, namely palmatine, was stored in the same conditions in the same humidity controlled enclosures having translucent walls at the same time and exposed to daylight in a laboratory. The dispensers were removed from the enclosures periodically and visually assessed, cap and former being replaced quickly after each observation. The visual change of the stick composition in the reference, acceptable, dispenser was compared with that observed in a dispenser that had a weight gain of 0.1846g after 21 days, i.e. a weight gain of 261% of the gain in the reference\dispenser.

In order to represent conditions during conventional use of a stick, the former was removed before the start of the trials.

At the commencement of the trials, the stick material was the same pale beige white in appearance. After 8 days storage, the stick composition in the reference dispenser was still pale beige white, whereas in the comparison dispenser the composition had darkened to beige. The stick composition in the reference dispenser did eventually darken to beige and observed after 16 days. This shows that the reference dispenser significantly retarded the rate at which the ingredient susceptible to photo-destruction was deleteriously affected and hence the reference dispenser is a suitable dispenser in accordance with the instant invention.

## Claims

1. A cosmetic product which is a composition disposed with a dispenser, in which the composition is in the form of a solid anhydrous stick composition containing one or more ingredients that are susceptible to photo-destruction by visible/UV light and/or UV light and the dispenser comprises a barrel having opposed first and second ends, the first end being closed by a cap and the second end comprising a base, a platform intermediate between the first and second ends and screw-threaded means for advancing the platform towards the first end mounted on or adjacent to the second end, the composition being disposed between the platform and the first end of the barrel, in which the dispenser permits a % weight gain after 21 days in the test procedure as defined herein of not greater than 150% of the % weight gain obtained in the reference dispenser.

2. A cosmetic product according to claim 1 in which the ingredient that is susceptible to photo-destruction by visible/UV light is one or a mixture selected from:-
Molecules which produce singlet oxygen under exposure to light in the range 290-740nm, preferably up to 700nm and oxygen with a quantum yield greater than 0.05 in an organic solvent;
Molecules comprising aromatic heterocycles
Phenols which lack a tertiary butyl group that would serve to stabilise the phenol, biological molecules: Vitamins, Co-enzymes, Enzymes and proteins and
Unsaturated aliphatic olefinically unsaturated fatty carboxylic acids

3. A cosmetic product according to claim 2 in which the ingredient that is susceptible to photo-destruction by visible/UV light is selected from:-
Aromatic heterocylic compounds, comprising a nitrogen hetero atom and particularly when the hetero atom is charged,
Unsaturated aliphatic olefinically unsaturated fatty carboxylic acids comprising a C-H bond alpha to the olefinic unsaturation;
Phenols and polyphenols that are not sterically hindered and
Vitamins.

4. A cosmetic product according to any preceding claim in which the ingredient that is susceptible to photo-destruction by visible/UV light is one or more of palmatine, a C18 olefinically unsaturated carboxylic acid and a vitamin.

5. A cosmetic product according to claim 4 in which the ingredient that is susceptible to photo-destruction by visible/UV light is palmatine.

6. A cosmetic product according to claim 1 or 2 in which the ingredient that is susceptible to photo-destruction by visible/UV light is a dye.

7. A cosmetic product according to any preceding claim in which the ingredient that is susceptible to photo-destruction by visible/UV light has a peak extinction coefficient in the visible/UV light spectrum of at least 100 mol⁻¹cm⁻¹.

8. A cosmetic product according to any preceding claim in which the ingredient that is susceptible to photo-destruction by visible/UV light has a peak extinction coefficient in the visible/UV light spectrum of at least 500 mol⁻¹cm⁻¹.

9. A cosmetic product according to any preceding claim in which the ingredient that is susceptible to photo-destruction by visible/UV light has a peak extinction coefficient in the visible/UV light spectrum of at least 1000 mol⁻¹cm⁻¹.

10. A cosmetic product according to any preceding claim in which the ingredient that is susceptible to photo-destruction by visible/UV light is present at a concentration of at least 0.0001% by weight, and preferably at least 0.001% by weight of the stick composition.

11. A cosmetic product according to any preceding claim in which the stick composition comprises an astringent antiperspirant salt.

12. A cosmetic product according to claim 11 in which the astringent salt is an aluminium and/or zirconium chlorohydrate, optionally complexed.

13. A cosmetic product according to claim 12 in which the astringent salt is an aluminium chlorohydrate.

14. A cosmetic product according to claim 12 in which the astringent salt is an aluminium zirconium chlorohydrate complexed with glycine.

15. A cosmetic product according to any preceding claim in which the dispenser permits a % weight gain after 21 days in the test procedure as defined herein of not greater than 110% of the % weight gain obtained in the reference dispenser.

16. A cosmetic product according to any preceding claim in which the base of the dispenser defines a central aperture through which extends a rotatable mounting for a rotor on which is mounted a spindle to engage the platform, the mounting and base being spaced apart and bridged by two seals.

17. A cosmetic product according to claim 16 in which the mounting and base comprise opposed axially extending concentric walls that are bridged by one seal of the two seals that is an unbroken radial annular seal.

18. A cosmetic product according to claim 16 in which the mounting and the base comprises a radially-extending wall section, which wall sections are opposed, the wall section of the mounting being located axially below the wall section of the base, the wall sections being bridged by an unbroken axial seal.

19. A cosmetic product according to claim 16 in which the mounting and the base each comprises a radial wall section orthogonal to an axially extending wall, the respective radial walls being opposed, the mounting radial wall being located below the base radial wall and the respective axially extending walls being opposed and concentric, the mounting wall being located within the base axially extending wall, and an annular radial seal bridging the axially extending walls and an axial seal bridging the radial wall sections.

20. A cosmetic product according to any of claims 16 to 19 in which at least one of the seals is a claw seal.

21. A cosmetic product according to any preceding claim in which the platform comprises at least one channel through which stick material can be charged into the dispenser between the platform and the first end.
